# EUROPEAN PATENT APPLICATION

(11) **EP 2 147 686 A1**
(43) Date of publication of application: **27.01.2010**
(21) Application number: 09175058.8
(22) Date of filing: 30.09.2005
(51) Int. Cl.: A61L 12/08, A61L 12/14, C11D 3/00, A61K 9/00, A45C 11/00

(54) **Method for packaging contact lenses**

(30) Priority: 01.10.2004 US 615256 P
(62) Divisional of application: 05791749.4
(71) Applicant: Menicon Co., Ltd., Nagoya 460-0006 (JP)
(72) Inventor: Newman, Stephen, D., 469983, Bayshore Park (SG); Glasbey, Trevor, O., Helensvale, Queensland 4212 (AU)
(74) Representative: Freeman, Jacqueline Carol

(57) **Abstract**

An aqueous contact lens storage solution comprises a viscoelastic rheology modifier; and not more than 0.3% w/v of an alkali metal salt; the solution has a pH of between 6.0 and 8.0 and an osmolality of between 100 and 400 mOsm/kg, and the solution has viscoelastic properties, even if heat sterilized at 121 DEG C for at least 15 minutes. The viscoelastic rheology modifier may be selected from the following acids or their alkali metal salts: hyaluronic acid, poly(acrylic acid), crosslinked poly(acrylic acid), poly(methacrylic acid), carboxymethyl cellulose, alginic acid and mixtures thereof.; The alkali metal salt may be present in an amount of from 0.01 % to 0.3% w/v, and be selected from sodium chloride, sodium bicarbonate, sodium dihydrogen phosphate, disodium hydrogen phosphate, trisodium phosphate, sodium tetraborate, sodium citrate, sodium acetate, sodium lactate, potassium chloride, potassium bicarbonate, potassium dihydrogen phosphate, dipotassium hydrogen phosphate, tripotassium phosphate, potassium tetraborate, potassium citrate, potassium acetate, potassium lactate and mixtures thereof. The solution may be an inhomogeneous combination of a gel component and an additional liquid component. If so, the solution has a pH of between 6.0 and 8.0 and an osmolality of between 100 and 400 mOsm/kg when in a homogeneous form.

## Description

### BACKGROUND

The present invention relates to the field of contact lens packages, and specifically to the solution contained within a contact lens package.

Currently available contact lenses, particularly soft contact lenses, afford a greatly improved level of comfort throughout the wearing cycle than that of the previously available lenses. However, when a contact lens, particularly a previously unworn lens, is inserted into the eye, it will generally feel less comfortable on initial insertion into the eye, with the wearer being aware of a foreign body in the eye. This initial phase of discomfort generally passes after 10 or so minutes. The initial insertion of the lens may also induce reflex tearing, a natural reaction to the presence of a foreign body in the eye, where copious quantities of watery, hypotonic tears are produced in order to flush the foreign body (in this case, a contact lens) from the eye.

Clinically, this initial period of wear may also be characterized by the well established observation that on initial insertion, a contact lens will generally exhibit excessive movement on the eye. Within the first few minutes of wear, the movement will reduce until it reaches a steady state value.

Traditionally, the tear film was thought to consist of three distinct layers, an outermost lipid layer, an aqueous layer that makes up 90% of the tear film volume, and a mucin layer that coats the ocular surface. However, it is now recognized that rather than being three distinct layers, the tear film is actually a lipid layer and an aqueous phase with differing concentrations of mucin throughout.

Mucin, a long chain glycoprotein, is secreted by specialized corneal cells (the goblet cells), whereas the lipid is secreted by the meibomian glands found on the lid margins. The aqueous phase of the tear film is secreted by the lachrymal glands, and is essentially filtered blood serum.

At the bottom of the tear film, the mucin gel phase is stabilized by the presence of corneal glycocalyx, which is secreted by the corneal epithelial cells. The presence of the glycocalyx assists in the binding of mucin to the normally hydrophobic corneal surface, a process which is also assisted by the presence of microvilli or protrusions found on the corneal epithelial cells.

The presence of mucin in the aqueous phase also gives rise to a viscoelastic rheology to the tear film. In open eye conditions, the tear film will exhibit a relatively high viscosity, which helps maintain a stable film over the cornea. However, when subjected to a sheer force during blinking, the tear film viscosity significantly reduces, which serves to lubricate the eyelids as they travel over the ocular surface. (See A J Bron, Prospects for the Dry Eye; Trans. Ophthalmol. Soc. UK 104 (1985) 801-826.) Another viscoelastic rheology modifier found naturally in the tear film is hyaluronic acid, a glycosaminoglycan polysaccharide.

Any deficiency in the tear film structure may result in dry eyes. For instance, damage to the corneal epithelial cells will result in a loss of the microvilli and also the glycocalyx. This may then produce hydrophobic spots on the cornea which lead to tear film instability, clinically manifested by a low tear break-up time. Such damage to the corneal epithelium is commonly caused by infections or trauma resulting in corneal scarring or ulcerations. Such ulcerations may occasionally result from wearing non-wetting contact lenses, or by hypoxic stress caused by wearing contact lenses during sleep.

Similarly, a reduction in the lipid layer will lead to the tear film being subject to excessive evaporation of the aqueous components of the tear film, which in turn leads to drying of the ocular surface. This evaporation may also lead to hypertonic stress on the epithelial goblet cells, which in turn will lead to a lowering of the mucin content of the tear film, again resulting in tear film instability.

While dry eye may contraindicate the wearing of contact lenses in the more severe cases, many asymptomatic, or marginally dry eye, patients will experience dry eye symptoms while wearing contact lenses. For these patients, the use of lubricating drops or wetting solutions may provide relief from the symptoms of dry eye, and allow a normal lens wearing period to be achieved.

Paradoxically, while daily disposable contact lenses offer the wearer a fresh lens each day, Solomon et al (CLAOJ, 1996; 22:250-257) has published data to suggest that symptoms of dryness among wearers of daily disposable lenses are more prevalent than in wearers of frequent replacement lenses, that is in lenses worn more than one day.

Therefore, there is a need to improve the comfort of a daily disposable lens on initial insertion into the eye, since an immediately comfortable lens would offer a marked advantage to the contact lens wearer.

### BRIEF SUMMARY

It has been discovered that the solution in which a contact lens is packaged can be used to impart needed lubricity to the lens. In a first aspect the invention is an aqueous contact lens storage solution comprising a viscoelastic rheology modifier; and not more than 0.3% w/v of an alkali metal salt; wherein said solution has a pH of between 6.0 and 8.0 and an osmolality of between 100 and 400 mOsm/kg, and wherein the solution has viscoelastic properties even if heat sterilized at 121°C for at least 15 minutes.

In another aspect, the invention is an aqueous contact lens storage solution which may be heat sterilized at 121°C for at least 15 minutes comprising 1) a viscoelastic rheology modifier selected from the following acids or their alkali metal salts: hyaluronic acid, poly(acrylic acid), crosslinked poly(acrylic acid), poly(methacrylic acid), carboxymethyl cellulose, alginic acid and mixtures thereof; and 2) from 0.01% to 0.3% of an alkali metal salt selected from sodium chloride, sodium bicarbonate, sodium dihydrogen phosphate, disodium hydrogen phosphate, trisodium phosphate, sodium tetraborate, sodium citrate, sodium acetate, sodium lactate, potassium chloride, potassium bicarbonate, potassium dihydrogen phosphate, dipotassium hydrogen phosphate, tripotassium phosphate, potassium tetraborate, potassium citrate, potassium acetate, potassium lactate and mixtures thereof. The solution has a pH of between 6.0 and 8.0 and an osmolality of between 100 and 400 mOsm/kg.

In another aspect, the invention is a contact lens package that contains a contact lens, a gel comprising viscoelastic rheology modifier and separate liquid component containing additional contact package solution components.

The packaging solution of the preferred embodiments of the invention provides for a lubricious, comfortable contact lens. In particular, the present invention is useful in providing an immediately comfortable lens, and in reducing the lens equilibration period following initial insertion.

### DETAILED DESCRIPTION OF THE PRESENTLY PREFERRED EMBODIMENTS

The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

As used herein, the term "contact lens storage solution" includes solutions that are intended for use in storing contact lenses, and include solutions used in contact lens packages, which are also referred to as "packaging solutions." Solutions that are intended for temporary contact with contact lenses, such as wetting solutions, cleaning solutions and eye drops are not included in the term "contact lens storage solution."

The packaging solution may be premixed as a homogeneous combination of ingredients when it is added to the package or, as explained in detail below, it may be made by placing two or more different components into the package which together form the packaging solution. In addition, part of the packaging solution may come from the lens itself. For example, an ocular lubricant included in the solution (or a portion thereof may be derived from the contact lens. It is common practice to add non-polymerizable substances such as glycerol or propylene glycol to a contact lens formulation as a processing aid, or non-reactive diluent, which remain within the unhydrated lens following polymerization. Once the lens has been fabricated, the non-reactive diluent will serve to plasticise the xerogel lens, and may render it soft and pliable. Typically, the diluent is then removed from the lens by extraction during lens hydration. If, however, the lens is hydrated in its final packaging, the diluent will extract from the lens and form part of the packaging solution.

According to a specific embodiment of this invention, the proposed packaging solution contains a viscoelastic rheology modifier, such as a polyanionic polymer, capable of imparting a viscoelastic rheology to the solution. Typically the concentration of the rheology modifier will be between 0.05% and 2%w/v, and more preferably between 0.1% and 1% w/v. If the viscoelastic rheology modifier is a polyanionic polymer, the polyanionic polymer may also serve as an ocular lubricant. As this invention is directed towards a packaging solution for contact lenses, it is also a requirement that the described solutions be suitable for terminal sterilisation by autoclaving at 121°C for at least 15 minutes and still maintains sufficient viscoelastic properties so as to provide the aforementioned lubricity when the lens is removed from the package.

By viscoelastic rheology, it is understood that the solution will exhibit non-Newtonian rheological behaviour, specifically a relatively high viscosity under conditions of low sheer and low viscosity when subjected to a high sheer force. Under zero or low shear (0.3 rpm) conditions, the solution viscosity will be in excess of 10 centipoise (cps), more preferably greater than 50 cps, and most preferably between 100 and 500cps; but under conditions of high shear (12 rpm), will fall below 25cps, and may fall below 2.5cpd, as measured by a Brookfield viscometer. The viscosity of preferred solutions at low shear will preferably be at least 150 %, and more preferably over 200%, and possibly even 300%, 500% or 1000% of the viscosity at high shear. Viscosity is measured at room temperature unless indicated otherwise.

Thus, in one aspect, the solution viscosity will preferably be in excess of 10cps under zero or low shear conditions (0.3 rpm), more preferably between 10 and 50cps, but under conditions of high shear (12 rpm), will fall below 2.5cps, as measured by a Brookfield viscometer.

In another aspect, the solution viscosity will preferably be in excess of 50cps under zero or low shear conditions (0.3 rpm), more preferably between 100 and 500cps, but under conditions of high shear (12rpm), will fall below 25cps, as measured by a Brookfield viscometer.

The viscoelastic rheology modifier may be selected from the following acids or the alkali metal salts thereof: hyaluronic acid, poly(acrylic acid), crosslinked poly(acrylic acid), poly(methacrylic acid), carboxymethyl cellulose, alginic acid and mixtures thereof. The preferred cross linked poly(acrylic acid) is carbomer, such as Carbopol® NF 941, Carbopol® NF 934 and other Carbopol varieties listed in U.S.P. 24 and National Formulary 19.

The viscoelastic rheology modifier may be a polyanionic polymer that is either fully or partially neutralised. Where the polyanionic polymer is partially neutralised, it may also act as a pH buffer.

Where a polysaccharide derivative, such as hyaluronic acid, is used as the viscoelastic rheology modifier in the solution, the average molecular weight of the polysaccharide derivative will preferably be of at least million Daltons and of low polydispersity prior to autoclaving in order to allow for any molecular weight reduction caused by hydrolysis during autoclaving without destroying the viscoelastic property of the solution. The effects of autoclaving on a hyaluronic acid solution have been discussed by Bothner and Waaler, Int. J. Biol Macromol (1988), 10: 287-291, incorporated herein by reference.

The solution may also contain up to about 0.3%w/v of an alkali metal salt. When the salt is included, it will preferably be used at a level of between about 0.01% and about 0.3%w/v of the storage solution. When used, more preferably it will comprise at **least 0.015%w/v** of the solution. Suitable alkali metal salts include sodium chloride, sodium bicarbonate, sodium dihydrogen phosphate, disodium hydrogen phosphate, trisodium phosphate, sodium tetraborate, sodium citrate, sodium acetate, sodium lactate, potassium chloride, potassium bicarbonate, potassium dihydrogen phosphate, dipotassium hydrogen phosphate, tripotassium phosphate, potassium tetraborate, potassium citrate, potassium acetate, potassium and mixtures thereof. Preferably the solution will contain sodium chloride, in a concentration between 0.01 and 0.3% w/v, preferably between 0.01 and 0.2% w/v, along with additional non-ionic tonicity adjusting agents.

These non-ionic tonicity agents must be non-toxic and non-irritating to the eye. Nonlimiting examples of non-ionic tonicity agents include simple sugars (for instance glucose, fructose, lactose, galactose, sucrose, mannose, xylose, ribose, arabinose, and sucrose), dexpanthenol, sorbitol, mannitol, ascorbic acid, urea, and arabitol. Those skilled in the art will recognize there are numerous other suitable examples. Most preferably, the additional non-ionic tonicity adjusting agents may also serve as an ocular lubricant, examples of which include glycerol, propylene glycol, polyethylene glycol, poly(glyceryl methacrylate), poly(vinyl alcohol), poly(vinyl pyrrolidinone) and mixtures thereof. The total concentration of these ocular lubricants should be between 0.1% and 5%, preferably between 0.2% and 2%. The tonicity adjusting agents, both alkali metal salts and non-ionic tonicity adjusting agents, may be used individually or in combination, to provide for a total solution tonicity in the range 100―400 mOsmol/kg, more preferably 200 - 400 mOsmol/kg. Wetting agents, such as non-ionic surfactants, amphoteric surfactants, and mixtures thereof may be added.

The pH of the solution is held between 6.0 and 8.0, preferably between 7.0 and 7.5. The pH may be held in the desired range either by additional buffering agents, such as sodium phosphate, sodium borate or sodium bicarbonate, or by the use of the intrinsic buffering action provided by partially neutralizing the polyanionic polymer.

Preferably the packaging solution is free of animal proteins, which may provoke undesirable immunological reaction in some contact lens wearers.

The solution described above may be used in place of the traditional, buffered saline currently in widespread use for packaging contact lenses, particularly for daily disposable Contact lenses packaged in blisters. Examples of this packaging system are given in U.S. Patents No. Des. 435,966; No. 4,691,820; No. 5,467,868; No. 5,704,468 and No. 5,823,327, and in Patent Cooperation Treaty Patent Publication No. WO 97/20019, all of which are incorporated herein by reference.

Typically, the plastic boat or blister is filled with the desired amount of the inventive solution, and a contact lens is then placed into the solution. The blister is then closed by heat sealing a plastic laminated aluminium foil over the blister. Once closed, the blister and contents may be sterilized by autoclaving.
The sealed, sterilized package may then be stored until required.

Upon opening by the patient, the lens may be inserted into the eye using a normal lens insertion technique.

Another problem that faces many contact lens manufacturers who package lenses in blisters is the tendency for the lens to adhere strongly to the blister, often to the extent where the lens is flattened against the walls of the blister. This is highly inconvenient to the lens wearer, and attempt to remove the adherent lens may lead to the lens becoming torn, or suffer edge damage. Typically, prevention of this lens adherence is achieved by the addition of controlled amounts of surfactants to the packaging saline.

The lens adherence arises from the fact that many contact lens materials may undergo a surface inversion, and change their surface character from essentially hydrophilic to essentially hydrophobic. When facing a high energy environment, such as water or saline, the hydrophilic moieties (for instance hydroxyl groups) on the polymer chain will be oriented out of the lens bulk, thus providing a hydrophilic surface. When facing a low energy environment (for instance air, or a hydrophobic polymer such as polypropylene), the contact lens material will express hydrophobic moieties (for instance methyl groups, or the polymer main chain) at the interface.

It is during autoclaving, when the water conter of the contact lens is reduced, that the lens may become strongly adhered to the walls of the polypropylene blister via a hydrophobic interaction.

In a second embodiment of the invention, a gel comprising the polymeric components in a minimal quantity of water is prepared (hereafter referred to as the gel component). A controlled quantity of the gel component may then be placed in the base of a standard contact lens packaging blister and used to adhere (or locate) the contact lens to the base of the package. The remaining ingredients are later added as a solution (hereafter referred to as the liquid component) to the lens package which is then sealed and autoclaved.

Without being bound to any particular theory, by adhering the lens on the blister surface with the above described gel, it is believed that a buffer layer is formed between the lens and the blister wall, thus preventing the lens adherence, particularly during autoclaving. After autoclaving, the viscoelastic components diffuse away from the surface over the course of 24-48 hours and form a homogenous solution.

It has also been found that the use of the gel component assists in the robotic transfer of the lens to the blister by providing for a degree of stiction, or temporary adhesion, which assists in the removal of the lens from the transfer arm.

In a third embodiment of the present invention, the gel component of the solution is again prepared separately, and may then be used to locate the lens for packaging in a slim-line, retort-type package, designed to hold the lens in a flattened state in which the internal depth of the retort-type package is less than the overall natural sagittal depth of the contact lens. Such a package is described in Patent Cooperation Treaty Application Serial No. PCT/AU02/01105, designating the United States, as well as U.S. Patent Application Serial No. 10/789,961, both of which are incorporated herein by reference. Following the addition of the liquid component, the package may be sealed and then sterilized by autoclave. By virtue of the slim-line design of the packaging, the inventive solution will remain in an essentially inhomogenous form, thus providing for a differential in lens/packaging adhesion between the two lens faces.

For example, Patent Cooperation Treaty Application Serial No. PCT/AU02/01105 describes a package that consists essentially of two sheets of polypropylene laminated aluminum, heat sealed together to form a sachet. The overall thickness can then be below 0.5mm, with the lens held within the foil in an essentially flat configuration.

Using this package and contact lens package solutions of the prior art, the contact lens, along with a small volume of saline, is contained within the package. During the packaging operation, a pre-hydrated contact lens is placed front surface down onto the center of a pre-cut section of foil. A small quantity of saline (typically >0.5ml) is then placed into the exposed concave surface (or base curve) of the contact lens, and a second laminated foil is then placed over the lens and heat sealed onto the bottom foil. During this operation, the lens is deformed into essentially a flat configuration, and the saline placed into the lens base-curve is trapped within the package in intimate contact with the lens so as to retain its equilibrium hydration state.

This same package may be used with the present invention. By applying the gel components of the inventive solution to the center of the bottom foil, greater certainty in retaining the lens in its optimal position during the packaging process can be achieved. The gel also serves to hold the lens in a stable orientation that allows the remaining liquid component of the inventive solution to be placed into the concave surface of the lens.

Surprisingly, it has also been found that the use of the gel component of the inventive solution to adhere the lens onto the bottom foil at a desired location also serves to ensure that the lens is always presented to the wearer in the optimal orientation when the sachet (or retort) pack is opened.

Upon opening of the sachet pack, the lens will be found to adhere loosely to the foil that was placed over the base curve of the lens. This allows the wearer to pinch the lens off the foil and insert it into the eye, confident in the knowledge that the lens will be in the correct orientation (i.e. not inside out).

Without being bound by any particular theory, it is believed that due to the restricted volume of the sachet pack, coupled with the fact the lens is held essentially flat, and in intimate contact against the packaging foils, there is little opportunity for the gel component and the liquid component to form a homogenous and that the front surface of the lens will be held in contact with a solution enriched in the gel component, whereas the base curve will be held in contact with a solution enriched in the liquid component.

This inhomogeneity will lead to the front surface of the lends exhibiting essentially zero adhesion to the bottom foil, whereas the base curve will adhere weakly to the top foil, thus ensuring that on opening the sachet, the lens will always be presented to the user front surface up.

Although the base curve does adhere weakly to the top foil, it is thought that the adhesion force is provided by capillary action, rather than a hydrophobic interaction, and therefore is not strong enough to lead to any detrimental effects.

Because these capillary forces are weak, the lens will not be pulled flat onto the laminated foil, but rather will exhibit a slight puckering, due to the compression of the hemispherical lens. The puckering provides a convenient point to lightly pinch the lens off the foil.

This may be contrasted with a lens binding hydrophobically to a flat polypropylene surface, where all of lens surface is pulled flat against the polypropylene, leading to an increase in apparent lens diameter.

On removal from the package, the higher concentration of viscoelastic material on the front surface of the lens may also assist the wearer to orientate and insert the lens. When subjected to a sheer force, the viscoelastic solution will show a drop in viscosity, and may exhibit a more lubricious surface. However, when the lens is held stationary on the finger, the higher apparent viscosity under these no sheer conditions will serve to hold the lens in place on the fingertip, thus easing insertion into the eye.

### EXAMPLES

### 1. Viscoelastic solution of sodium hyalonurate (low salt formulation)

One liter of deionized water is placed in a cylindrical mixing vessel, and stirred at a moderate rate using a blade stiffer. Sodium chloride (0,10g), propylene glycol (10.00g) and arabitol (10.00g) are then added to the water and dissolved.

One gram of lyophilized sodium hyaluronate (m. weight 3,000,000 Daltons) is then slowly added portion-wise by sprinkling into the stirrer vortex. Stirring is continued for several hours until a clear, homogeneous viscoelastic solution is formed. The pH of the resultant solution is then adjusted to between 7.2 and 8.00 by the appropriate addition of 0.1M hydrochloric acid solution or 0.1M triethanolamine solution to give a solution of approximately 2.1mOsmol/kg.

### 2. Viscoelastic solution of sodium hyalonurate (high salt formulation)

One liter of deionized water is placed in a cylindrical mixing vessel, and stirred at a moderate rate using a blade stirrer. Sodium chloride (0.30g), propylene glycol (7.50g) and arabitol (10.00g) are then added to the water and dissolved.

100 ml of Healon® (Pfizer, Inc.), a 1% solution of sodium hyaluronate, is then added portion-wise into the stirred solution. Stirring is continued for several hours until a clear, homogenous viscoelastic solution is formed. The pH of the resultant solution is then adjusted to between 7.2 and 8.00 by the appropriate addition of 0.1M hydrochloric acid solution or 0.1M triethanolamine solution to give a solution that is approximately 242 mOsmol/kg.

### 3. Use of carbomer as viscoelastic rheology modifier

One liter of deionized water is placed in a cylindrical mixing vessel, and stirred at a moderate rate using a blade stirrer. Carbopol® NF 941 (1.50g) is then sprinkled portion-wise into the stirrer vortex over 30 minutes, and stirring maintained until a homogenous dispersion is achieved.

Sodium chloride (2.0g), propylene glycol (10.00g), glycerol (5.0g) and anhydrous glycose (10,00g) are then added sequentially to the water and dissolved.

0.1M Sodium hydroxide solution is then added drop-wise to the above turbid solution while stirring is continued, until the pH is between 7.0 and 7.6. During the addition of the sodium hydroxide the solution will clarify and a noticeable viscosity increase will be observed. The final osmolality of the solution will be approximately 309 mOsmol/kg.

### 4. Use of mixed viscoelastic rheology modifiers

One liter of deionized water is placed in a cylindrical mixing vessel, and stirred at a moderate rate using a blade stirrer. Carbopol® NF 941 (1.00g) is then sprinkled portion-wise into the stirrer vortex over 30 minutes, and stirring maintained until a homogenous dispersion is achieved. 50 ml of Healon®, a 1% solution of sodium hyaluronate (Pfizer Inc.), is then added portion-wise by into the stirred solution.

Sodium chloride (0.5g), glycerol (10.0g), glucose (10.00g) and arabitol (10.00g) are then added sequentially to the water and dissolved.

0.1M sodium hydroxide solution is then added drop-wise to the above turbid solution while stirring is continued, until the pH is between 7.0 and 7.6. During the addition of the sodium hydroxide the solution will clarify and a noticeable viscosity increase will be observed. The final osmolality of the solution will be approximately 250 mOsmol/kg.

### 5. Use of gel to adhere lens in a standard blister design

A small (0.05ml) droplet of Healon® (Pfizer Inc.) is placed onto the center of a standard polypropylene blister. A hydrated contact lens is then placed front surface down onto the Healon® droplet. 0.45 ml of an aqueous solution of sodium chloride (0.01% w/v), propylene glycol (0.75% w/w) and arabitol (1% w/w) is then added to the blister, which is then closed by heat sealing a second laminated foil onto the top of the polypropylene spacer.

The package may then be sterilized by autoclaving at 121°C for 15 minutes. The package is then held for 24 hours to allow the contents to reach equilibrium. On opening, the tonicity of the enclosed solution will be approximately 242 mOsmol/kg.

### 6. Derivation of a portion of the ocular lubricant from the lens.

A contact lens formulation is prepared by mixing 2-hydroxyethyl methacrylate (54,00g), glycerol monomethacrylate (35.00g), ethyleneglycol dimethacrylate (0.5g), glycerol (10g) and benzoin methyl ether (0.5g). The lens formulation is then dosed into a two part polypropylene mould, and polymerized by exposure to UV radiation (360nm). Following polymerization, the partially plasticized lens is removed from the mould. The mass of the unhydrated lens will be 20mg, of which 2mg will be glycerol. The dry lens is placed in a contact lens blister boat, and 0.5ml of the packaging solution from Example 1added. The blister is then closed, and the package autoclaved. The packaging solution will then have the following formulation: sodium chloride (0.01%), hyaluronic acid (0.1%),propylene glycol (1.0%), and glycerol (0.4%), of which, the glycerol is derived entirely from the unhydrated lens.

### 7. Use of gel to locate lens in a sachet style package

For the purposes of this example, the lens container is comprised of two laminated aluminium foils, heat sealed to form a sachet.

A small (0.05ml) droplet of Healon® (Pfizer Inc.) is placed onto the center of a pre-cut polypropylene laminated aluminium foil. A hydrated contact lens is then placed front surface down onto the Healon® droplet. 0.45 ml of an aqueous solution of sodium chloride (0.01%,w/v), propylene glycol (0.75% w/w) and arabitol (1% w/w) is then added to the base curve of the lens, and the package completed by heat sealing a second laminated foil onto the bottom foil.

The package may then be sterilized by autoclaving at 121°C for 15 minutes. Because of the reduced volume of entrapped air, a non-balanced autoclave may be successfully used to sterilize the package. On opening, the lens will be loosely bound base curve down, and in the correct orientation on the top foil. Furthermore, the lens will be found to have a slight ripple in the center, allowing the lens to be removed easily for insertion into the eye.

The use of a gel comprising a viscoelastic rheology modifier, and a separate liquid component containing additional contact package solution components, both contained in a contact lens package provides a unique contact lens package which allows for benefits in putting a lens into the package.

Various modifications and variations of the described methods and compositions of the invention will be apparent to those skilled in the art without departing from the scope and spirit of the invention. The independent claims that follow provide statements of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in chemistry or related fields are intended to be within the scope of the following claims.
Various combinations of features are listed below in numbered paragraphs for a better understanding of this technology:
1. An aqueous contact lens storage solution comprising:
   a) a viscoelastic rheology modifier; and
   b) not more than 0.3% w/v of an alkali metal salt;
   c) wherein said solution has a pH of between 6.0 and 8.0 and an osmolality of between 100 and 400 mOsm/kg, and wherein the solution has viscoelastic properties even if heat sterilized at 121°C for at least 15 minutes.
2. The contact lens storage solution of claim 1 wherein the rheology modifier is selected from the following group of acids and their alkali metal salts:
   hyaluronic acid, poly(acrylic acid), cross linked poly(acrylic acid), poly(methacrylic acid), carboxymethyl cellulose, alginic acid and mixtures thereof
3. The contact lens storage solution of any one of claims 1 and 2 comprising between about 0.01% and about 0.3% w/v of said alkali metal salt.
4. The contact lens storage solution of any one of claims 1 to 3 wherein said alkali metal salt is selected from the group consisting of sodium chloride, sodium bicarbonate, sodium dihydrogen phosphate, disodium hydrogen phosphate, trisodium phosphate, sodium tetraborate, sodium citrate, sodium acetate, sodium lactate, potassium chloride, potassium bicarbonate, potassium dihydrogen phosphate, dipotassium hydrogen phosphate, tripotassium phosphate, potassium tetraborate, potassium citrate, potassium acetate, potassium lactate and mixtures thereof.
5. An aqueous contact lens storage solution which may be heat sterilized at 121°C for at least 15 minutes comprising:
   a) a viscoelastic rheology modifier selected from the following group of acids and their alkali metal salts: hyaluronic acid, pply(acrylic acid), cross linked poly(acrylic acid), poly(methacrylic acid), carboxymethyl cellulose, alginic acid and mixtures thereof; and
   b) from 0.01% to 0.3% w/v of an alkali metal salt selected from the group consisting of sodium chloride, sodium bicarbonate, sodium dihydrogen phosphate, disodium hydrogen phosphate, trisodium phosphate, sodium tetraborate, sodium citrate, sodium acetate, sodium lactate, potassium chloride, potassium bicarbonate, potassium dihydrogen phosphate, dipotassium hydrogen phosphate, tripotassium phosphate, potassium tetraborate, potassium citrate, potassium acetate, potassium lactate and mixtures thereof;
   c) wherein said solution has a pH of between 6.0 and 8.0 and an osmolality of between 100 and 400 mOsm/kg.
6. The contact lens storage solution of any one of claims 1 to 5 wherein the rheology modifier comprises a polyanionic polymer.
7. The contact lens storage solution of any one of claims 1 to 6 wherein the rheology modifier comprises a polysaccharide derivative with an average molecular weight in excess of 1 million Daltons prior to sterilization by autoclaving.
8. The contact lens storage solution of any one of claims 1 to 7 wherein the solution has a viscosity in excess of 50cps when measured with a Brookfield viscometer at 0.3 rpm and a viscosity of less than 25cps when measured with a Brookfield viscometer at 12 rpm.
9. The contact lens storage solution of any one of claims 1 to 7 wherein the solution has a viscosity, after sterilization by autoclaving at 121°C for at least 15 minutes, in excess of 10cps when measured with a Brookfield viscometer at 0.3 rpm and a viscosity of less than 2.5cps when measured with a Brookfield viscometer at 12 rpm.
10. The contact lens storage solution of any one of claims 1 to 7 wherein the solution has a viscosity, after sterilization by autoclaving at 121°C for at least 15 minutes, in excess of 50cps when measured with a Brookfield viscometer at 0.3 rpm and a viscosity of less than 25cps when measured with a Brookfield viscometer at 12 rpm.
11. The contact lens storage solution of any one of claims 1 to 7 wherein the solution has a viscosity, after sterilization by autoclaving at 121°C for at least 15 minutes, of between about 100 and 500cps when measured with a Brookfield viscometer at 0.3 rpm and a viscosity of less than 25cps when measured with a Brookfield viscometer at 12 rpm.
12. The contact lens storage solution of any one of claims 1 to 11 Wherein the concentration of the rheology modifier is between 0.05% and 2% w/v.
13. The contact lens storage solution of any one of claims 1 to 11 wherein the concentration of the rheology modifier is between 0.1% and 1% w/v.
14. The contact lens storage solution of any one of claims 1 to 13 further comprising at least one additional ingredient selected from the group consisting of ocular lubricants, non-ionic tonicity adjusting agents, and wetting agents.
15. The contact lens storage solution of any one of claims 1 to 13 further comprising an ocular lubricant selected from the group consisting of glycerol, propylene glycol, polyethylene glycol, poly(glyceryl methacrylate), polyvinyl alcohol), polyvinyl pyrrolidinone) and mixtures thereof.
16. The contact lens storage solution of claim 15 wherein the ocular lubricant is present at a concentration of between 0.1% and 5%.
17. The contact lens storage solution of any one of claims 1 to 13 further comprising one or more non-ionic tonicity adjusting agents selected from the group consisting of glucose, fructose, lactose, galactose, sucrose, mannose, xylose, ribose, arabinose, sucrose, dexpanthenol, sorbitol, mannitol, ascorbic acid, urea, and arabitol, such that the final solution has an osmolality of between 200 and 400 mOsm/kg.
18. The contact lens storage solution of any one of claims 1 to 13 further comprising a agent selected from the group consisting of a non- ionic surfactant, an amphoteric surfactant, and mixtures thereof.
19. A method for packaging a contact lens in which a contact lens its immersed into the solution of any one of claims 1 to 1 in a container, the container sealed, and the contents sterilized by autoclaving.
20. The method of claim 19 wherein the molecular weight of the rheology modifier is in excess of 1 million Daltons prior to sterilization by autoclaving.
21. A method ofpackaging a contact lens in which a contact lens is immersed in a solution in a container, the container sealed, and the contents sterilized by autoclaving, wherein the solution is an inhomogeneous solution that contains a viscoelastic rheology modifier which is added to the container as part of a concentrated gel and additional ingredients; and wherein, after sealing the container and autoclaving the contents, a solution according to any one of claims 1 to 18 is formed.
22. The method of any one of claims 19 to 21 wherein the container is a glass vial.
23. The method of any one of claims 19 to 21 wherein the container is a plastic blister.
24. The method of claim 23 where the blister has a depth of less than 5mm.
25. The method of claim 23 where the blister has a depth greater than the natural sagittal height of the contact lens.
26. The method of claim 23 wherein the blister has a depth substantially smaller the natural sagittal height of the contact lens.
27. The method of any one of claims 19 to 26 wherein the solution comprises an ocular lubricant extracted from the contact lens.
28. The method of claim 21 where the gel is used to adhere a contact lens to an inside surface of the container prior to the addition of the remaining solution ingredients.
29. The method of claim 21 wherein the application of viscoelastic rheology modifier as a concentrated gel substantially prevents the lens from becoming strongly bound to a wall of the container.
30. The method of any one of claims 21 and 28 to 29 wherein the packaging solution remains essentially inhomogenous after the autoclaving.
31. The method of claim 30 wherein the inhomogeneity provides a differential adhesion strength of the front surface of the lens to the container compared to the adhesion of the back surface of the lens to the container.
32. The method of claim 31 wherein the differential adhesion strength provides for the presentation of the contact lens in a controlled orientation when the container is opened.
33. The method of claim 32 where the lens face contacting the gel when the lens is placed in the container is exposed when the container is opened.
34. A contact lens package that contains
   a) a contact lens and
   b) an aqueous contact lens storage solution which, if homogeneously mixed, comprises:
      i) a viscoelastic rheology modifier; and
      ii) not more than 0.3% w/v of an alkali metal salt;
      iii) and said solution has a pH of between 6.0 and 8.0 and an osmolality of between 100 and 400 mOsm/kg, and wherein the solution has viscoelastic properties even if heat sterilized at 121°C for at least 15 minutes.
35. A contact lens package that contains an aqueous contact lens storage solution which may be heat sterilized at 121°C for at least 15 minutes, the storage solution, if homogeneously mixed, comprising:
   a) a viscoelastic rheology modifier selected from the following group of acids and their alkali metal salts: hyaluronic acid, poly(acrylic acid), crosslinked poly(acrylic acid), poly(methacrylic acid), carboxymethyl cellulose, alginic acid and mixtures thereof; and
   b) from 0.01% to 0.3% w/v of an alkali metal salt selected from the group consisting of sodium chloride, sodium bicarbonate, sodium dihydrogen phosphate, disodium hydrogen phosphate, trisodium phosphate, sodium tetraborate, sodium citrate, sodium acetate, sodium lactate, potassium chloride, potassium bicarbonate, potassium dihydrogen phosphate, dipotassium hydrogen phosphate, tripotassium phosphate, potassium tetraborate, potassium citrate, potassium acetate, potassium lactate and mixtures thereof;
   c) wherein said solution, if combined into a homogeneous solution, has a pH of between 6.0 and 8.0 and an osmolality of between 100 and 400 mOsm/kg.
36. The contact lens package of any one of claims 34 and 35 wherein the solution comprises a gel component comprising said viscoelastic rheology modifier and a separate liquid component.
37. A contact lens package that contains a contact lens, a gel comprising viscoelastic rheology modifier and separate liquid component containing additional contact package solution components.

## Claims

1. A method of packaging a contact lens in which a contact lens is immersed in a solution in a container, the container sealed, and the contents sterilized by autoclaving, wherein the solution is an inhomogeneous solution that contains a viscoelastic rheology modifier which is added to the container as part of a concentrated gel and additional ingredients; and wherein, after sealing the container and autoclaving the contents, a solution is formed which comprises not more than 0.3% w/v of an alkali metal salt; and has a pH of between 6.0 and 8.0 and an osmolality of between 100 and 400 mOsm/kg, and wherein the solution has viscoelastic properties.

2. The method of claim 1 wherein the container has a depth substantially smaller than the natural sagittal height of the contact lens.

3. The method of any one of claims 1 to 2 wherein the solution comprises an ocular lubricant extracted from the contact lens.

4. The method of any one of the preceding claims where the gel is used to adhere a contact lens to an inside surface of the container prior to the addition of the remaining solution ingredients.

5. The method of any one of the preceding claims wherein the packaging solution remains essentially inhomogenous after the autoclaving.

6. The method of claim 5 wherein the inhomogeneity provides a differential adhesion strength of the front surface of the lens to the container compared to the adhesion of the back surface of the lens to the container; wherein the differential adhesion strength provides for the presentation of the contact lens in a controlled orientation when the container is opened.

7. The method of any one of the preceding claims wherein the rheology modifier is selected from the following group of acids and their alkali metal salts: hyaluronic acid, poly(acrylic acid), cross linked poly(acrylic acid), poly(methacrylic acid), carboxymethyl cellulose, alginic acid and mixtures thereof.

8. The method of any one of the preceding claims wherein the solution comprises between about 0.01% and about 0.3% w/v of said alkali metal salt.

9. The method of any one of the preceding claims wherein said alkali metal salt is selected from the group consisting of sodium chloride, sodium bicarbonate, sodium dihydrogen phosphate, disodium hydrogen phosphate, trisodium phosphate, sodium tetraborate, sodium citrate, sodium acetate, sodium lactate, potassium chloride, potassium bicarbonate, potassium dihydrogen phosphate, dipotassium hydrogen phosphate, tripotassium phosphate, potassium tetraborate, potassium citrate, potassium acetate, potassium lactate and mixtures thereof.

10. The method of any one of the preceding claims wherein the rheology modifier comprises a polyanionic polymer.

11. The method of any one of the preceding claims wherein the rheology modifier comprises a polysaccharide derivative with an average molecular weight in excess of 1 million Daltons prior to sterilization by autoclaving.

12. The method of any one of claims 1 to 11 wherein the solution has a viscosity, after sterilization by autoclaving at 121°C for at least 15 minutes, in excess of 10cps when measured with a Brookfield viscometer at 0.3 rpm and a viscosity of less than 2.5cps when measured with a Brookfield viscometer at 12 rpm.

13. The method of any one of claims 1 to 11 wherein the solution has a viscosity, after sterilization by autoclaving at 121°C for at least 15 minutes, in excess of 50cps when measured with a Brookfield viscometer at 0.3 rpm and a viscosity of less than 25cps when measured with a Brookfield viscometer at 12 rpm.

14. The method of any one of claims 1 to 11 wherein the solution has a viscosity, after sterilization by autoclaving at 121°C for at least 15 minutes, of between about 100 and 500cps when measured with a Brookfield viscometer at 0.3 rpm and a viscosity of less than 25cps when measured with a Brookfield viscometer at 12 rpm.

15. The method of any one of the preceding claims wherein the concentration of the rheology modifier is between 0.05% and 2% w/v.

16. The method of any one of the preceding claims wherein the additional ingredients are selected from the group consisting of ocular lubricants, non-ionic tonicity adjusting agents, and wetting agents.

17. The method of any one of the preceding claims further comprising an ocular lubricant selected from the group consisting of glycerol, propylene glycol, polyethylene glycol, poly(glyceryl methacrylate), polyvinyl alcohol), polyvinyl pyrrolidinone) and mixtures thereof.

18. The method of any one of the preceding claims further comprising one or more non-ionic tonicity adjusting agents selected from the group consisting of glucose, fructose, lactose, galactose, sucrose, mannose, xylose, ribose, arabinose, sucrose, dexpanthenol, sorbitol, mannitol, ascorbic acid, urea, and arabitol, such that the final solution has an osmolality of between 200 and 400 mOsm/kg.

19. The method of any one of the preceding claims wherein the molecular weight of the rheology modifier is in excess of 1 million Daltons prior to sterilization by autoclaving.

20. The method of any one of the preceding claims wherein the solution comprises the gel component comprising said viscoelastic rheology modifier and a separate liquid component.

21. A contact lens package formed by the method of any one of claims 1 to 20.
